# EUROPEAN PATENT APPLICATION

(11) **EP 4 159 206 A1**
(43) Date of publication of application: **05.04.2023**
(21) Application number: 20937806.6
(22) Date of filing: 31.08.2020
(51) Int. Cl.: A61K 9/56, A61K 31/4985, A61P 15/10, A61P 13/08

(54) **LONG-LASTING REABSORBABLE SUBCUTANEOUS IMPLANT WITH SUSTAINED RELEASE OF PRE-CONCENTRATED PHARMACOLOGICALLY ACTIVE SUBSTANCE IN POLYMER FOR THE TREATMENT OF ERECTILE DYSFUNCTION AND BENIGN PROSTATIC HYPERPLASIA**

(30) Priority: 29.05.2020 BR 102020010933
(71) Applicant: Luiz Peracchi, Edson, 81230-161 Curitiba (BR)
(72) Inventor: Luiz Peracchi, Edson, 81230-161 Curitiba (BR)
(74) Representative: De Pablos Riba, Juan Ramon
(86) International application number: PCT/BR2020/050346
(87) International publication number: WO 2021/237322

(57) **Abstract**

The present application for priority of invention is directed to the medical sector and comprises a biodegradable implant containing tadalafil in a polymer matrix. This implant is inserted into the subcutaneous layer of the patient and has continuous release of the pharmaceutical active over an extended period of time. The release aims to achieve an efficient and prolonged serum level of pharmacy for the treatment of erectile dysfunction and benign prostatic hyperplasia. The implant may have in its constitution only tadalafil, but is preferably formed by tadalafil particles dispersed homogeneously in a biocompatible and biodegradable polymer matrix. The polymer matrix may be formed by only one polymer or by a mixture of polymers. The implants have a shape and size that facilitates their insertion into the patient and provides a prolonged and uniform release of the active ingredient contained. The drug is released constantly, throughout the treatment period. Furthermore, the implant can contain a polymer coating wall and, as such, the duration of the treatment can vary between 3 and 6 months, thereby configuring the proposed period of time between the insertions of new implants.

## Description

The present priority of invention application is directed to the medical sector and comprises a long-lasting resorbable subcutaneous implant with prolonged release of a pharmacologically active substance encased in polymer for treatment of erectile dysfunction and benign prostatic hyperplasia.

### STATE OF THE ART

One of the treatments used for Erectile Dysfunction (ED) and Benign Prostatic Hyperplasia (BPH) and the use of tadalafil, a reversible and selective inhibitor of the 5-phosphodiesterase enzyme. ED is defined as the recurrent inability to obtain and maintain an erection of the genital organ that allows satisfactory sexual activity and is more common in men from the age of 40. It is not exactly a disease, but rather a manifestation of symptoms of one or more pathologies. The normal erection depends on the relaxation of the smooth muscle of the corpus cavernosum, the increase of the arterial blood flow in and the restriction of the venous outflow. Changes in this system end up interfering negatively in the erection mechanism and culminating in ED. Examples of pathologies that can interfere with erections are systemic arterial hypertension, diabetes mellitus, metabolic syndrome, smoking, dyslipidemia, neurological diseases, hormonal disorders and chronic use of certain drugs. Furthermore, other causes include depression, psychiatric disorders, relationship conflicts and performance anxiety during sexual intercourse.

Referring to BPH, pathological evidence usually appears in men between 40 and 50 years and tend to advance from the first and second to the third stage as age increases. BPH is the increase in the number of cells in this region of the bladder/urethra. There is an imbalance between the amount of cells that are born and the amount of cells that die, thereby producing an increase in volume in the region capable of causing pressure on the bladder and urethra. Benign prostatic growth is not directly related to cancer and diagnosis is performed through clinical and laboratory tests of histology and pathology. BPH causes patients to report symptoms in the lower urinary tract (LUTS), which include difficulty in controlling urination, forcing while urinating, weak or dripping urinary flow and a feeling of incomplete urination.

For the exclusive treatment of ED, there is the pharmaceutical form in powder intended for injection of alprostadil (prostaglandin analog E1) 10 mcg or 20 mcg and ampoule with 1 mL of water for injection to be applied directly to the penis immediately before the sexual act. The effect usually takes 20 minutes after application and lasts a maximum of 2 hours, depending on the patient. It is an uncomfortable and difficult route of administration for the patient, resulting in fibrosis, bleeding and hematoma at the site of application or maintenance of the erection for an undesirably long time. Another point to consider is the fact that this drug requires storage in a refrigerator, for this reason the patient should take additional care.

However, intracavernous injection is not advantageous, as it is necessary that the physician and patient to be willing to spend a period (days or weeks) adjusting the application dose, and there is also the need for patient training in this procedure. Furthermore, the patient must carry a syringe and needle to do the application before the sexual act or interrupt the act to apply the drug. [007] A few years ago, the treatment of choice for BPH adopted alpha-blockers such as tamsulosin and terazosin. These drugs have certain side effects such as nausea, low blood pressure and sexual dysfunction. It is known that about 70% of patients with BPH also have ED. For this reason, in addition to sexual dysfunction, alpha-blockers have been replaced by 5-phosphodiesterase inhibitors (IPDE5) for the treatment of BPH and ED, considerably improving the patient's quality of life.

Tadalafil, sildenafil and vardenafil belong to the class of 5-phosphodiesterase (IPDE5) enzyme inhibitors. These drugs inhibit the action of the enzyme 5-phosphodiesterase, allowing the increase of the intracellular concentration of guanosine triphosphate cyclase (cGMP), which promotes the relaxation of the smooth muscle of the corpus cavernosum (erectile structure of the penis) and dilation of the arteries responsible for taking the blood to the organ. Thus, the blood flow in the region is facilitated, producing an erection and reducing the symptoms of BPH.

Sildenafil is commercially available in pharmaceutical form as 25 mg, 50 mg and 100 mg coated tablets and vardenafil in the same pharmaceutical form, but only at 20 mg. Meanwhile, tadalafil is found in coated tablets of 5 mg and 20 mg.

Tadalafil is the only 5-phosphodiesterase enzyme inhibitor available at a low dosage of 5 mg and is the form of daily oral administration able to treat ED and HPB concomitantly. Tadalafil acts both in patients with ED or BPH and in the two associated pathologies. The improvement in quality of life when using tadalafil is a consequence of the few adverse effects observed, as there are no reports of hypotension or the possibility of sexual dysfunction occurring during its use.

In addition, the daily oral administration of tadalafil allows the patient to approach a natural sex life, since this drug has a half-life of 17.5 hours (4.38 times longer than the half-life of sildenafil, which is 4 hours). Thus, a daily administration of tadalafil 5 mg is sufficient for the patient to enjoy its effect at any time throughout a full day.

Another positive point of tadalafil and its high efficacy and safety even at higher doses and in patients difficult to treat, such as those who have diabetes mellitus. The recommended dose is 5 mg daily or a single dose of 20 mg orally prior to sexual intercourse. The daily administration of tadalafil is preferred by 72% of patients, besides being the most indicated form of treatment for quern desires a behavior closer to an active and natural sexual life. Similarly, daily treatment with tadalafil for 12 weeks results in significant clinical reduction of BPH symptoms.

The disadvantages of the oral route for the treatment of any disease are related to patient compliance, first-pass metabolism in the liver, and partial loss of the drug through the absorptive process. The success or failure of drug therapy, in this case, depends completely on the patient. Therapeutic adhesion is impacted by the frequency of administration. Medication administered in a single dose provide greater adhesion when compared to those administered in multiple doses. Oral treatment is the most susceptible to failure, as it is dependent on the active participation (or compliance) of the patient and, in addition, it involves socioeconomic factors, factors related to the health team and the disease itself. Compliance is "participatory, active obedience by the patient with the medical prescription", with the prescription understood as not only the medication, but also of all other care or measures recommended by the physician or other health professional.

Treatment adherence rates are generally higher among patients with acute conditions, compared to those in chronic conditions, and dramatically reduces after the first six months of therapy when it comes to chronic conditions. Patients generally improve the behavior of taking medication in the previous five days and after a consultation with the physician, compared to the past thirty days, in a phenomenon known as "white coat compliance".

When asked about the reasons why patients do not take their medication strictly, the typical reasons cited are forgetfulness, other priorities, decision to omit doses, lack of information and emotional factors. Other factors that influence the lack of adherence to treatment are related to complex prescriptions, lack of knowledge about the benefits and side effects of the drug, incompatibility of the pharmaceutical form with the patient's lifestyle or the cost of the drug.

In general, patients do not adhere to treatment in 50% of the time and, more specifically in the case of tadalafil, where the target is men, this percentage increases even more. This group is the least adherent to medical guidance. There is a clinical concern, as younger men have the lowest rate of adherence to treatment.

The oral route is propitious for the incorrect use of medication, increasing the risks of underdoses or supra-physiological doses. In these two situations, the patient is at risk of recurrence of symptoms, which could compromise the general condition of the patient. It should be noted that patients affected by chronic pathologies often discontinue treatment when they notice the reversal of symptoms or when they feel some discomfort due to side effects or adverse reactions, becoming more susceptible to a worsening of their clinical condition.

From the state of the art related to the use of tadalafil, the patent records BR 10 2017 025256-6, BR 10 2017 021360-9, BR 10 2015 015988-9, BR 11 2016 010411-0, BR 11 2015 032728-1, PI 1006266-1, PI 0618385-9, PI 0621852-0, PI 0606122-2 and PI 0313484-9 can be cited. All records mention the use of tadalafil, but the release of the substance is carried out by means of oral tablets, injectables with scheduled IM release, transdermal, topical, cream, ovules, spray, aerosol, liquid, solution, gel and ointment, and there is no record that adopts a long-lasting resorbable subcutaneous implant with sustained release.

The most effective way to increase the therapeutic adhesion of the patient and simplify the use of the medication as much as possible, from reducing the number of times the patient needs to go to the drugstore to acquire the medication to the pharmaceutical form, dose, route of administration, the flavor of the medication, reducing the number of administrations, the occurrence of side effects and preventing polypharmacy.

Considering this scenario and seeking to overcome the low therapeutic adhesion of patients, avoiding self-medication, we have the pharmaceutical form known as bioabsorbable implants or pellets with doses very close to the physiological dose. The implants present sustained release of the pharmaceutical active to treat the pathologies in the most physiological way and thus avoid the appearance of adverse effects. This pharmaceutical form is efficient in ensuring the patient's adherence to treatment and, consequently, the success of such, since the frequency in the application of new implants is staggered and it is not necessary to intervene the patient, nor is it necessary to remove the pellet at the end of the active release period, since when implanted in the patient's body, the resorbable subcutaneous implants release drugs by a prolonged period, giving convenience to travel and reducing the frequency of administration, as these devices allow a personalized medicine and treatment with release in weeks, months or years in a device that can be removed, if adverse effects require interruption of treatment.

The terms "implant" or "pellet" refer to this pharmaceutical form already consolidated in the official collections of standards for pharmaceutical drugs and substances. They are solid and sterile preparations of size and format suitable for subcutaneous implantation and release of the active substance(s) over an extended period of time.

Biodegradable or bioerodible implants can be understood as implants carrying some mechanism that gradually reduces their mass for an extended period of release time. Furthermore, it is possible for them tor erode and their components to gradually dissolve. The terms also refer to total degradation and absorption by the organism occurring at the site where the implants were applied, excluding the need to remove the implants at the end of the treatment. This mechanism of release of the active principle through the implant is due to the composition of this pharmaceutical form, the polymers.

The terms "prolonged release", "slow release" or "sustained release" refer to the speed/form of release of the drug from the implant, which happens continuously and gradually for a period of extended time and does not result in an immediate and concentrated release of the drug in the body.

Biodegradable polymers or bioerodible polymers are polymers that degrade *in vivo* and their erasure, over time, simultaneously and/or subsequently affects the release of the therapeutic agent. A biodegradable polymer may be a homopolymer, copolymer, or a polymer compressing more than two polymer units. In some cases, a biodegradable polymer can include mixing two or more homopolymers or copolymers.

Compiling the deficiencies and pre-existing problems regarding the existing pharmaceutical forms to treat ED and BPH, the present invention aims at the use of the previously detailed polymer subcutaneous implant, promoting the prolonged release of tadalafil directly into the bloodstream at low doses, without first-pass metabolism in the liver, thereby minimizing unwanted side effects and offering the patient a better quality of life. Another advantage of the present invention is the elimination of residues in the tissues after the end of the treatment, which rules out the need for surgeries to remove the implant. A third advantage of the invention concerns the release of the drug in very low doses, being very close to a normal physiological condition. Thus, the patient with ED can have a normal sex life, without the disorders observed when using the single-dose tablet or intracavernous application.

### DESCRIPTION OF THE FIGURES

For a better understanding of the present invention, the following drawings are attached:
Figure 1 - Representation of the chemical structure of the tadalafil compound;
Figures 2 and 3 - Dimensional designs of tadalafil implants;

### DETAILED DESCRIPTION OF THE INVENTION

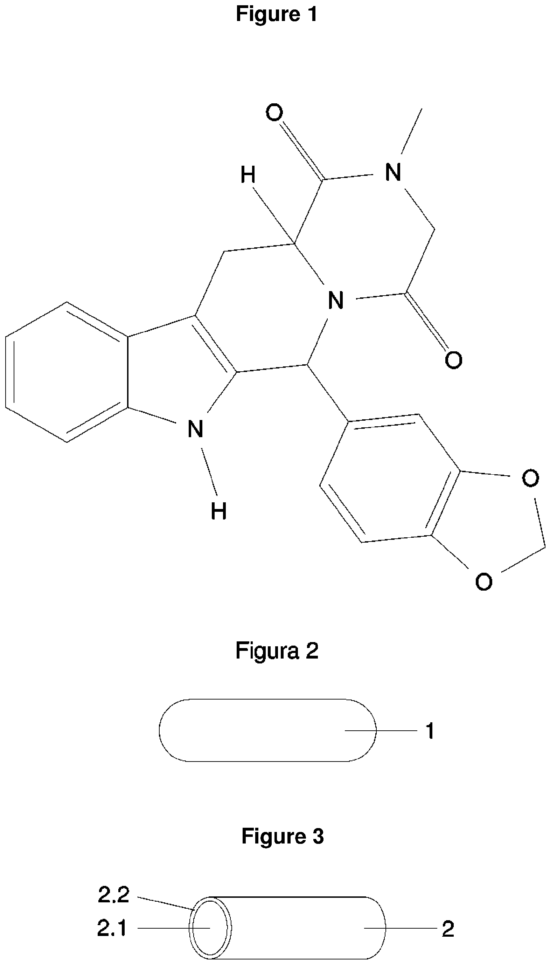

The present application for priority of invention is summarized in a biodegradable implant containing tadalafil in a polymer matrix. The implant is inserted subcutaneously and has a continuous release of the active substance for a prolonged period of time. This release aims to ensure an efficient serum level of the drug for a long time for the treatment of ED and BPH. The drug refers to tadalafil, i.e., an active molecule that inhibits the enzyme 5-phosphodiesterase. Its chemical structure is shown in figure 1.

The proposed bioadsorbable implant may have in its constitution only tadalafil, but is preferably formed by tadalafil particles dispersed homogeneously in a bioerodible and bioabsorbable polymer matrix. Such a polymer matrix may be formed of a polymer or a mixture of polymers. The amount of tadalafil in the implant can range from 25 to 250 mg per implant and its composition can have from 1 to 20% biodegradable polymer in relation to its weight of tadalafil. Preferably it should have from 20 to 110 mg and from 2 to 10% of biodegradable polymer in proportion to weight, forming a homogeneous mixture.

The biodegradable polymer used may be: Poly(D-lactic acid), Poly(L-lactic acid), Poly(racemic lactic acid), Poly(glycolic acid), Poly(caprolactone), methylcellulose, ethylcellulose, hydroxypropyl cellulose (HPC), hydroxypropyl methylcellulose (HPMC), polyvinylpyrrolidone (PVP), poly(vinyl alcohol) (PVA), polyethylene 6-oxide) (PEO), polyethylene glycol, starch, wax, natural gum and synthetic gum.

Implants can have any size, shape or structure that facilitates their manufacture and subcutaneous insertion in order to obtain a more constant and uniform release of the active ingredient and to obtain geometric shapes that remain over time.

Thus, the implant developed and exposed in the present application adopts the cylindrical pattern (1), rod-shaped with straight or rounded tips and length between 2 to 25 mm and diameter of 1 to 6 mm. An approximate illustration of the biodegradable implant can be seen in figure 2.

The implant manufacturing process starts with the addition of 25 to 250 mg of tadalafil in the polymer matrix chosen in the proportion of 1 to 20% in relation to the weight of the drug, in its dry forms, in powder. The mixture is then added into a container to be homogenized. If the polymer solvent is not also tadalafil solvent, it will be dispersed in the form of particles or suspension, and a mixer can be used to make the solution homogeneous. Then, this solution is dried and later molded to the shape of the implant (1) or other desired shape.

The mixture of actives in the manufacture of the implant can be molded by pressure or heat, so as not to compromise the effectiveness and chemical structure of tadalafil, nor to degrade the polymer. The options of techniques for molding the implant can be: injection molding, hot molding, compression molding or extrusion molding.

For the present invention, the technique chosen was compression molding. In this technique, the homogenized powder actives are added in a mold and there is the application of mechanical force under the mixture, compressing the particles and molding the implant. Then there is the filling and sterilization of the implant containing tadalafil. Sterilization, in this case, is done by exposing the contents to 90 °C in an oven for 1 hour.

The implant may have a coating polymer membrane, with a thickness that may range from 0.1 to 0.7 mm. The polymer used for the coating must be bioabsorbable and allow the passage of the active substance. The coating of the implant is preferably done by dipping the implant in a polymer solution. The coating may cover the entire surface of the implant including the edges, only its longitudinal surface with the edges uncoated or coated only on the edges of the implant without coating its length. The polymers that can be used for the coating are: co-glycolic polylactic acid (PLGA) and D,L-lactic acid copolymers.

Another type of implant option for treatment of erectile dysfunction and benign prostatic hyperplasia are non-biodegradable implants. Non-biodegradable or non-bioerodible implants (2) (figure 3) have a central core (2.1) formed by a polymer matrix in the percentage of 1 to 20% in relation to the weight of the drug, in this case 25 to 250 mg of tadalafil, with the core being surrounded by a non-degradable polymer membrane (2.2) that controls the release rate of the drug.

The polymer membrane manufacturing material surrounding the implant may be: silicone, urethane, acrylates and their copolymers, polyvinylidene fluoride copolymers, ethylene polyethylene vinyl acetate-vinyl, dimethylpolysiloxane. This membrane has a thickness of 0.2 to 1 mm and is molded by specific equipment. After molding the membrane from the polymer material, the active mixture is inserted, forming the central core (2.1) of the implant (2). The polymers used in the polymer matrix and the mixture adopt the same compounds and process as the bioabsorbable implant.

The release of the drug in this system occurs through diffusion, at a relatively constant rate, and it is possible to change the speed of release of the drug through the thickness or material of this membrane. In this system, there is a need to remove the implant at the end of the treatment.

The proposed implant has a duration of approximately 3 to 6 months, and this is the period proposed between the insertions of the implants. The complete treatment lasts according to remission of symptoms of ED and/or symptoms in the lower urinary tract from BPH. The therapeutic window of tadalafil in the scientific literature, orally and 2.5 mg or 5 mg once a day, depends on drug tolerance in order to achieve clinical results in the treatment of ED. In oral administration there is a loss of active principle due to first-pass metabolism, as well as the loss of part of the active due to the absorptive process, added these losses can reach 50% of the drug ingested. The implant has a release of 0.6 to 2.0 mg of tadalafil per day, depending on the amount of drug present in it. In practice with the use of 2-4 implants, with concentrations between 25 and 250 mg, the expected results are consolidated.

However, to define an individualized treatment for each patient, the physician must evaluate the clinical condition of this patient, use his laboratory tests as support for the decision making of the dosage and, based on the daily release of tadalafil by the implants, begin supplementation with partial doses. After this first evaluation, the dose can be adjusted by inserting additional implants, if necessary. Furthermore, if there is a rejection or any adverse reaction after insertion of the implant, it can be removed within the first days of treatment. If there is no rejection, removal of the implant is not necessary after the treatment period, since it leaves no residues in the tissues because they are fully absorbed by the body.

The use of the implant proposed herein is safe and effective in the treatment of ED and BPH with symptoms in the lower urinary tract. This is because the therapy does not depend on patient discipline for maintaining the dosage, and there is regularity of the treatment due to the low release of the active principle through slow but stable doses, promoting the patient quality of life through a close to natural sexual life while at the same time producing an interval in the frequency of application of new implants.

It should be noted once again that patients affected by chronic diseases often discontinue treatment when they notice the reduction of symptoms or when they feel some discomfort due to adverse reactions, becoming more susceptible to a worsening of their clinical condition.

Thus, the use of implants in drug therapy avoids discontinuity and ensures proper treatment, as well as the effectiveness of such. In the case of tadalafil, patients who use the biodegradable pellet will have more convenience in performing any daily activity without worrying about the administration of the tablet or if the drug is stored in the refrigerator or if they are carrying accessories for intracavernous application of the drug. The advantages of using the bioabsorbable implant guarantee the patient a practically normal routine in life.

Another benefit of the invention is the release of the drug directly into the bloodstream, which makes its action much more efficient, avoiding gastrointestinal metabolism and the effect of the first hepatic passage of the drug, as it is released directly into the systemic circulation, improving the bioavailability of the drug. Thus, the dose required for treatment can be reduced, minimizing unwanted side effects and ensuring the therapeutic adherence of the patient.

## Claims

1. "Long-lasting resorbable subcutaneous implant with prolonged release of a pharmacologically active substance preconcentrated in polymer for the treatment of erectile dysfunction and benign prostatic hyperplasia", **characterized by** the biodegradable implant containing tadalafil in a bioerodible and bioabsorbable polymeric matrix in particle form, with the amount of tadalafil in the implant varying from 25 to 250 mg and the biodegradable polymer composition being 1 to 20% in relation to the weight in tadalafil;

2. "Long-lasting subcutaneous reabsorbable implant with prolonged release of a pharmacologically active substance pre-concentrated in polymer for the treatment of erectile dysfunction and benign prostatic hyperplasia", according to claim 1, **characterized by** the biodegradable polymer used being Poly(D-lactic acid), Poly(L-lactic acid), Poly(racemic lactic acid), Poly(glycolic acid), Poly(caprolactone), methylcellulose, ethylcellulose, hydroxypropyl cellulose (HPC), hydroxypropyl methylcellulose (HPMC), polyvinylpyrrolidone (PVP), poly(vinyl alcohol) (PVA), polyethylene 6-oxide) (PEO), polyethylene glycol, starch, wax, natural and synthetic gum;

3. "Long-lasting subcutaneous reabsorbable implant with prolonged release of pharmacologically active substance pre-concentrated in polymer for the treatment of erectile dysfunction and benign prostatic hyperplasia", according to claims 1 and 2, **characterized by** the implant adopting the cylindrical pattern (1), in the shape of a rod with straight or rounded tips and length between 2 to 25 mm and diameter of 1 to 6 mm;

4. "Long-lasting subcutaneous reabsorbable implantwith prolonged release of a pharmacologically active substance pre-concentrated in polymer for the treatment of erectile dysfunction and benign prostatic hyperplasia", according to claims 1, 2 and 3, **characterized by** the implant manufacturing process starting with the addition of 25 to 250 mg of tadalafil in the polymer matrix chosen in the proportion of 1 to 20% in relation to the weight of the drug, in its dry forms, in powder, with the addition of the mixture in a container to be homogenized, with the insertion of the active powder in a mold to be performed molding the implant under mechanical force, compressing the particles and molding the implant in the format (1), with the filling and sterilization of the implant at 90 °C in an oven for 1 hour;

5. "Long-lasting subcutaneous reabsorbable implant with prolonged release of a pharmacologically active substance pre-concentrated in polymer for the treatment of erectile dysfunction and benign prostatic hyperplasia", according to claims 1, 2 and 3, **characterized by** the fact that the implant has a polymer membrane coating, with a thickness between 0.1 to 0.7 mm, with total coating of the implant including the edges, only its longitudinal surface coated with the edges uncoated or coated only on the edges of the implant without coating its length, using as co-glycolic polylactic acid (PLGA) and copolymers of acid D,L-lactic as the polymer membrane;

6. "Long-lasting subcutaneous reabsorbable implant with prolonged release of a pharmacologically active substance pre-concentrated in polymer for the treatment of erectile dysfunction and benign prostatic hyperplasia", **characterized by** the fact that the implant is presented in non-biodegradable or non-bioerodible form (2), having a central core (2.1) formed by a polymer matrix in the percentage of 1 to 20% in relation to the weight of the drug, in this case 25 to 250 mg of tadalafil, with the core surrounded by a non-degradable polymer membrane (2.2);

7. "Long-lasting subcutaneous reabsorbable implant with prolonged release of a pharmacologically active substance pre-concentrated in polymer for the treatment of erectile dysfunction and benign prostatic hyperplasia", according to claim 6, **CHARACTERIZED by** the fact that the manufacturing material of the POLYMER membrane that surrounds the non-biodegradable implant is silicone, urethane, acrylates and their copolymers, copolymers of polyvinylidene fluoride, polyethylene vinyl acetate-vinyl ethylene and dimethylpolysiloxane, with said membrane having a thickness of 0.2 to 1 mm and the insertion of tadalafil occurring after its molding, forming the central core (2.1) of the implant (2).
